Europäisches Patentamt

**European Patent Office**

Office européen des brevets

(19)

(11) **EP 1 353 287 A1**

(12) **EUROPÄISCHE PATENTANMELDUNG**

(43) Veröffentlichungstag:
**15.10.2003 Patentblatt 2003/42**

(51) Int Cl.⁷: **G06F 19/00**

(21) Anmeldenummer: **02008042.0**

(22) Anmeldetag: **10.04.2002**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(71) Anmelder:
• **SIEMENS AKTIENGESELLSCHAFT**
**80333 München (DE)**
• **Dr. Hein GmbH**
**90429 Nürnberg (DE)**

(72) Erfinder:
• **Abraham-Fuchs, Klaus**
**91058 Erlangen (DE)**

• **Eisermann, Uwe**
**91052 Erlangen (DE)**
• **Hein, Achim**
**90403 Nürnberg (DE)**
• **Richter, Nils**
**95349 Thurnau (DE)**
• **Setz, Robert**
**91126 Rednitzhembach (DE)**

(74) Vertreter: **Berg, Peter, Dipl.-Ing.**
**European Patent Attorney,**
**Siemens AG,**
**Postfach 22 16 34**
**80506 München (DE)**

(54) **Verfahren und System zur Messung des Behandlungserfolges einer medizinischen Therapie**

(57) Die vorliegende Erfindung betrifft ein Verfahren sowie ein System zur Messung des Behandlungserfolges einer medizinischen Therapie. Bei dem Verfahren wird an einer ersten Datenverarbeitungsstation (10) zumindest ein Behandlungsziel festgelegt und eine Berechnungsvorschrift für den Behandlungserfolg der Therapie bereitgestellt, wobei die Berechnungsvorschrift den Behandlungserfolg als Funktion erfassbarer Maßzahlen angibt. Weiterhin wird eine zweite Datenverarbeitungsstation (20) zur automatisierten Erfassung und Übermittlung der den Behandlungserfolg beschreibenden Maßzahlen an die erste Datenverarbeitungsstation (10) bereitgestellt. Zu Beginn der Therapie wird der Ausgangszustand des Patienten in Bezug auf das Behandlungsziel mit ersten Maßzahlen erfasst. Während und/oder am Ende der Behandlung werden automatische den Behandlungserfolg beschreibende zweite Masszahlen durch die zweite Datenverarbeitungsstation (20) erfasst. Anhand dieser Maßzahlen wird der Behandlungserfolg automatisch berechnet und an der ersten (10) oder einer weiteren Datenverarbeitungsstation dargestellt. Das Verfahren ermöglicht eine zuverlässige und automatisierte Messung des Behandlungserfolges einer medizinischen Therapie, insbesondere im Bereich telemedizinischer Behandlungsformen, und lässt sich vorteilhaft in den normalen Therapieablauf einbetten.

EP 1 353 287 A1

**Beschreibung**

Die vorliegende Erfindung betrifft ein Verfahren sowie ein System zur Messung des Behandlungserfolges einer medizinischen Therapie oder Therapiemaßnahme, wie sie insbesondere im Rahmen einer Rehabilitation oder in Zusammenhang mit Disease Management Services für Patienten mit chronischen Krankheiten durchgeführt wird. Ein besonderes Anwendungsfeld stellen telemedizinische Behandlungsformen dar, bei denen der Patient zur Therapie gehörende Trainingseinheiten in seinem häuslichen Umfeld durchführt und lediglich über eine Datenverbindung mit dem betreuenden Arzt oder Therapeuten in Verbindung steht.

Neuere Entwicklungen im Gesundheitswesen, insbesondere die evidenzbasierte Medizin, medizinische Guidelines und die Einführung des DRG-Systems (Disease Related Groups), führen verstärkt zu dem Wunsch nach einer objektiven Messbarkeit des Erfolges einer medizinischen Behandlung. Durch eine derartige Messung des Behandlungserfolges, im Folgenden auch als Outcome bezeichnet, kann eine Entscheidungshilfe für die weitere Vorgehensweise bei der Behandlung erhalten werden. Eine standardisierte Messung des Behandlungserfolges kann auch für den Vergleich der Qualität unterschiedlicher medizinischer Leistungserbringer herangezogen werden.

Bisher wird üblicherweise der Behandlungserfolg einer medizinischen Therapie nur im Rahmen klinischer Studien, nicht jedoch in der Alltagsroutine der medizinischen Versorgung gemessen. Außerdem erfolgt bei derartigen Studien in der Regel eine Einschränkung auf überschaubare Teilaspekte der Behandlung, wie bspw. auf den Vergleich zweier Medikationsschemata oder auf den Vergleich zweier chirurgischer Vorgehensweisen.

Ein Verfahren zur konsistenten vergleichbaren und mittels Computerunterstützung automatisierten Messung des Behandlungserfolges einer medizinischen Therapie, das sich in den üblichen klinischen Arbeitsablauf bzw. den Disease Management Service einbetten lässt, ist bisher nicht bekannt. Der Bedarf an einer derartigen Messung wird jedoch zunehmend größer, da sich vermehrt neue telemedizinische Behandlungsformen durchsetzen, die mit Schlagwörtern wie Telemedizin, Homecare und integrierte Versorgung umschrieben werden. Telemedizinische Behandlungsformen sind sowohl für die Nachbetreuung nach Rehabilitationsmaßnahmen als auch bei chronischen Erkrankungen wie bspw. Diabetes, Asthma oder Herzinsuffizienz besonders geeignet. Sie sind in der Regel durch die kooperative Beteiligung mehrerer medizinischer Leistungserbringer in einem Behandlungsprozess, die Komplexität und die lange Dauer des Behandlungsprozesses charakterisiert. Die Behandlung kann sich hierbei über einen sehr langen Zeitraum von Monaten bis Jahren erstrecken. In der Behandlung müssen häufig Multimorbiditäten, d. h. das gleichzeitige Vorliegen mehrerer Erkrankungen, berücksichtigt werden, so dass sich die Behandlung aus vielen, nur scheinbar unabhängigen Teilprozessen bzw. Maßnahmen zusammensetzt. Eine Messung des Behandlungserfolges durch separate Messung des Outcome der einzelnen in sich scheinbar geschlossenen Teilprozesse ist zwar organisatorisch einfacher, führt jedoch häufig vom Ziel der Messung des Behandlungserfolges der Gesamttherapie weg, da in vielen Fällen die Summe der separat gemessenen Erfolge der Einzelmaßnahmen kein zuverlässiges Maß für den Erfolg der Gesamttherapie darstellt. Unter Therapie ist hierbei die Summe der einzelnen Therapiemaßnahmen zu verstehen, aus denen sich die Behandlung zusammensetzt.

Die Aufgabe der vorliegenden Erfindung besteht darin, ein Verfahren sowie ein System zur Messung des Behandlungserfolges einer medizinischen Therapie anzugeben, die sich in den normalen Behandlungsprozess integrieren lassen und eine vergleichbare und zuverlässige Messung des Behandlungserfolges der gewählten Therapie, insbesondere bei einem räumlich verteilten telemedizinischen Rehabilitations- oder Behandlungsprozess, ermöglichen.

Die Aufgabe wird mit dem Verfahren sowie dem System gemäß den Patentansprüchen 1 bzw. 22 gelöst. Vorteilhafte Ausgestaltungen des Verfahrens sowie des Systems sind Gegenstand der Unteransprüche oder lassen sich der nachfolgenden Beschreibung und den Ausführungsbeispielen entnehmen. Anspruch 2 gibt ein das Verfahren gemäß Anspruch 1 untersützendes Verfahren zur Bestimmung des Behandlungserfolges an.

Bei dem vorliegenden Verfahren zur Messung des Behandlungserfolges einer medizinischen Therapie wird zunächst zumindest ein Behandlungsziel der Therapie festgelegt. Hierbei handelt es sich bevorzugt um ein der Summe aller Therapiemaßnahmen der Therapie übergeordnetes Ziel, wie bspw. die verbesserte oder aufrechterhaltene Lebensqualität des Patienten, die Selbständigkeit der Lebensführung, einen verringerten Grad der Berufsunfähigkeit usw.. Anschließend wird eine Berechnungsvorschrift für den Behandlungserfolg der Therapie in Bezug auf das Behandlungsziel bereitgestellt, die den Behandlungserfolg als Funktion erfassbarer Maßzahlen angibt. Die Maßzahlen stellen hierbei vorzugsweise nicht absolute physikalische Größen sondern relative Größen dar, wie bspw. den Prozentsatz der Zielerreichung, den Bruchteil der Zielerreichung oder eine normierte Punktezahl (Score). Sie können einzelne Messwerte darstellen oder aus ein oder mehreren Messwerten abgeleitet sein. Es versteht sich von selbst, dass diese Maßzahlen - oder die Größen, aus denen sie berechnet werden - zuverlässig erfassbar bzw. messbar sein müssen. Schließlich werden den Ausgangszustand des der Therapie zu unterziehenden Patienten in Bezug auf das Behandlungsziel beschreibende ein oder mehrere erste Maßzahlen vorzugsweise zu Beginn der Therapie erfasst. Die erste Maßzahl kann bspw. den Messwert für den Grad eines bestehenden Defizits zum Anfang der Behandlung in relativen Maßeinheiten bezogen auf eine Referenzgröße oder auf die Größe des Defizits bei Erreichen des Behandlungsziels darstellen. Das festgelegte Behandlungsziel, die gewählte Berechnungsvorschrift sowie die ersten Maßzahlen werden schließlich abgespeichert. Dies kann in einer entsprechend dafür vorgesehenen Datenbank oder auch in einer elektronischen Patientenakte erfolgen. Die Festlegung des Behandlungsziels sowie das Bereitstellen der Berechnungsvorschrift wird an einer ersten Datenverarbeitungsstation durchgeführt, an der dem Therapeuten oder Arzt die entsprechenden Mittel zur Verfügung stehen. Weiterhin wird dem Patienten eine zweite Datenverarbeitungsstation zur Verfügung gestellt und vorzugsweise derart konfiguriert, dass sie die den Behandlungserfolg beschreibenden Maßzahlen im Verlauf der Therapie Computer-unterstützt und weitgehend automatisiert erfassen kann. Für die Berechnung des Behandlungserfolges zu einem beliebigen Zeitpunkt im Verlauf der Behandlung oder zum Ende der Behandlung werden durch die zweite Datenverarbeitungsstation das betrachtete Defizit des Patienten zu diesem Auswertungszeitpunkt beschreibende zweite Maßzahlen erfasst und über ein Netzwerk an die erste Datenverarbeitungsstation übermittelt. An dieser oder der zweiten Datenverarbeitungsstation werden die vorher abgespeicherten Daten abgerufen. Auf der Basis dieser Daten, d. h. zumindest des Behandlungsziels, der Berechnungsvorschrift, der ersten sowie der zweiten Maßzahlen, erfolgt die automatische Berechnung und Darstellung des Behandlungserfolges mit der Berechnungsvorschrift. Die Berechnung kann an der ersten Datenstation sowie auch bereits an der zweiten Datenstation erfolgen, von der dann zusätzlich oder anstelle der zweiten Maßzahlen der berechnete Behandlungserfolg an die erste Datenverarbeitungsstation übermittelt wird.

Selbstverständlich lassen sich beim vorliegenden Verfahren auch mehrere Behandlungsziele vorgeben, die sich auf einzelne Therapiemaßnahmen oder Gruppen von Therapiemaßnahmen beziehen können. In diesem Fall müssen möglicherweise für jedes Behandlungsziel eine andere Berechnungsvorschrift bereitgestellt und andere erste und zweite Maßzahlen erfasst werden. Die Berechnungsvorschrift kann beim vorliegenden Verfahren neben einer Kategorie von Maßzahlen für das medizinische Ergebnis E der Behandlung, ausgedrückt durch die obigen ersten und zweiten Maßzahlen, auch von weiteren Kategorien abhängig sein. Derartige Kategorien umfassen bspw. Maßzahlen für die Kosten K der Behandlung, eine Maßzahl für die Behandlungsdauer T und eine Maßzahl für die Compliance C des Patienten. Die Berechnungsvorschrift kann somit eine allgemeine Funktion der mathematischen Form $f(E, K, C, T)$ darstellen. Die Maßzahlen können bspw. auch tabellenartige Punktebewertungen (Scores) sein, bei denen das Behandlungsergebnis durch die Summation von Punkten erzielt wird, die die Maßzahlen für die Kategorien E, K, T und C repräsentieren. Eine Zuordnung der Punkte zu den gemessenen Werten kann bspw. über sog. Lookup-Tables erfolgen.

Im Folgenden werden das vorliegende Verfahren sowie bevorzugte Ausführungsformen dieses Verfahrens anhand einer konkreten Berechnungsvorschrift nochmals verdeutlicht. Es versteht sich jedoch von selbst, dass für die Berechnung des Behandlungserfolges aus den entsprechenden Maßzahlen auch andere Berechnungsvorschriften zum Einsatz kommen können, die den Behandlungserfolg als Funktion der entsprechenden Maßzahlen angeben.

Eine wichtige Voraussetzung für die Messung des Behandlungserfolges ist die klare und eindeutige Beschreibung eines Behandlungsziels, wobei der Grad der Erreichung dieses Behandlungsziels messbar sein muss. In diesem Zusammenhang kann es von Vorteil sein, mehrere Teilziele anzugeben, die in verschiedenen Stufen des Behandlungsprozesses zu erreichen sind. Durch die Wahl relativer Maßzahlen beim vorliegenden Verfahren wird eine Vergleichbarkeit der Erreichung der einzelnen Teilziele gewährleistet.

Wichtige Behandlungsziele einer Therapie, die der Summe aller Therapiemaßnahmen übergeordnete Ziele darstellen, sind im Folgenden beispielhaft nicht abschließend aufgezählt. Ein häufiges Ziel einer rehabilitativen Therapie oder Therapiemaßnahme ist die Minderung oder Beseitigung eines krankheitsoder unfallbedingten Defizits in der körperlichen und geistigen Leistungsfähigkeit. Diese Defizite sind in grober Regel die Folge der Schwächung oder des kompletten Ausfalls einer Gehirnregion, eines Muskels oder eines Organs. Ein Fähigkeits- oder Leistungsdefizit kann in Prozent des Ausfalls der 100 %igen Leistungsfähigkeit einer durchschnittlichen gesunden Normalperson gemessen werden. Die messbare Fähigkeit kann bspw. die Beweglichkeit eines Gelenkes in Grad, die Kraft eines Muskels in Kilogramm Belastbarkeit oder die Größe des Gesichtsfeldes in Grad bei Sehstörungen betreffen. Ein weiteres Behandlungsziel kann die Verbesserung von Fertigkeiten sein, die insbesondere für die selbständige Versorgung des Patienten wichtig sind. Unter einer Fertigkeit wird im Kontext einer medizinischen Rehabilitationsmaßnahme eine komplexe Handlung verstanden, die aber in sich geschlossen und gegenüber anderen Handlungen abgrenzbar ist. Für eine Fertigkeit wird das Zusammenspiel mehrerer Fähigkeiten benötigt. Ein weiteres Ziel einer rehabilitativen Maßnahme kann ganz allgemein die Verbesserung der Lebensqualität des Patienten sein. Für die Messung der Lebensqualität gibt es Messinstrumente in Form von standardisierten Fragebögen, die vom Patienten ausgefüllt werden. Ein anderes klar definiertes Ziel einer Behandlung kann auch die Verbesserung des Zustands des Patienten im Hinblick auf eine Pflegebedürftigkeit sein, so dass der Patient bei Erreichen des Behandlungsziels in eine bessere Pflegestufe eingeordnet werden kann. Die Zuordnung zu einer Pflegestufe wird im Allgemeinen durch die Bewertung mehrerer Fertigkeits- und Fähigkeits-Defizite bestimmt. Für den Patienten bedeutet eine verbesserte Pflegestufe ein selbstbestimmteres Leben, für das Gesundheitssystem geringere Pflegekosten. Ein anderes Behandlungsziel kann die Reduktion des Grades einer bestehenden Berufsunfähigkeit sein, der bspw. in Prozent der Berufsunfähigkeit als Maßzahl angegeben werden kann, um eine weitestmögliche Wiedereingliederung in das Berufsleben zu ermöglichen. Ein weiteres Behandlungsziel kann die Verbesserung oder Stabilisierung einer Organfehlfunktion sein, die sich durch Messung der Abweichung eines physiologischen Parameters von einem gesunden Normalbereich quantifizieren lässt, bspw. durch die Messung des Blutdruckes bei einer Erkrankung des Herz-Kreislaufsystems, durch die Messung der Herzrate unter definierter Belastung bei Herzinsuffizienz oder durch Atemvolumen-Messungen bei Asthma.

Nach der Festlegung des Behandlungsziels der Therapie wird für den Patienten eine Datenbank mit dem Behandlungsziel oder den Behandlungs-Teilzielen, zumindest einem Messwert für einen Grad zumindest eines bestehenden Defizits D zum Anfang. der Behandlung in relativen Maßeinheiten und der gewählten Berechnungsvorschrift für den Behandlungserfolg angelegt. Optional kann auch ein erwarteter Zielwert des Defizits nach erfolgtem Abschluss der Behandlung als Prognose mit abgespeichert werden. Zumindest am Ende der Behandlung wird ein weiterer Messwert für das Defizit D in relativen Maßeinheiten erfasst und für die Berechnung des Behandlungserfolges herangezogen. Als Berechnungsvorschrift kann hierbei beispielhaft eine der folgenden Formeln für das qualitäts-orientierte Outcome (Q) dienen:

$$\text{Outcome (Q)} = \text{Ergebnis} = (1 - \text{Defizit}), \text{also:}$$

$$\text{Outcome (Q)} = 1 - D\ (\text{Ende}), \text{oder}$$

$$\text{Outcome (Q)} = D\ (\text{Anfang}) - D\ (\text{Ende})$$

In einer vorteilhaften Ausführungsform des vorliegenden Verfahrens wird der Messwert für das Defizit D nicht nur am Anfang und am Ende der Behandlung, sondern in beliebig vorgebbaren Zeitabständen bzw. zu beliebig vorgebbaren Zeitpunkten Ti im Verlauf der Behandlung in relativen Maßeinheiten erfasst. Auf diese Weise lässt sich die Veränderung gegenüber dem Ausgangszustand zu den vorgegebenen Zeitpunkten während der Behandlung darstellen. Aus diesen Zwischenwerten des Outcome kann auch eine Verlaufskurve erzeugt werden, die dem Therapeuten bzw. Arzt Aufschluss über den zeitlichen Verlauf des Behandlungserfolges gibt.

Die Erfassung bzw. Messung der jeweiligen Maßzahlen kann durch unterschiedliche Messtechniken bzw. Mittel erfolgen, die von der Art des mit der Maßzahl zu beschreibenden Defizits abhängen. So lassen sich die Messwerte für die Maßzahlen bspw. durch Ausfüllen eines entsprechenden Fragebogens durch den Patienten oder durch Messung einer Trainingsleistung mit einem entsprechenden Messwertaufnehmer am Trainingsgerät erfassen. Diese Mittel sind mit der zweiten Datenverarbeitungsstation verbunden, die am Ort des Trainings bzw. der Therapiemaßnahmen, insbesondere in der häuslichen Umgebung des Patienten, aufgestellt und mit der ersten Datenverarbeitungsstation des Therapeuten oder Arztes zumindest zeitweise über ein Netzwerk verbunden ist. Die zweite Datenverarbeitungsstation ist als Computerarbeitsplatz ausgestaltet und derart konfiguriert bzw. mit Mitteln ausgestattet, dass sie die für die Erfassung der Maßzahlen erforderlichen Messungen automatisch zu den vorgebbaren Zeitpunkten durchführt oder veranlasst und an die erste Datenverarbeitungsstation übermittelt. In umgekehrter Richtung lässt sich auch die Aufnahme der erforderlichen Messwerte an der zweiten Datenverarbeitungsstation durch die erste Datenverarbeitungsstation veranlassen. Die zweite Datenverarbeitungsstation kann bei bestimmten Behandlungsformen auch beispielsweise durch einen Palmtop realisiert sein, falls sich dieser für die Erfassung der entsprechenden Maßzahlen eignet.

Unter dem steigenden Kostendruck im Gesundheitswesen ist es für einen medizinischen Leistungserbringer in zunehmendem Ausmaß nicht mehr ausreichend, alleine das Erreichen des medizinischen Zieles im Auge zu haben. Vielmehr muss dass Erreichen des medizinischen Ergebnisses in Relation zu den hierzu eingesetzten Ressourcen, d. h. der verursachten Kosten, gesehen werden. In der bevorzugten Ausführungsform des vorliegenden Verfahrens wird daher eine Berechnungsvorschrift für ein kostenorientiertes Outcome (E,K) bereitgestellt, bei dem das erreichte Behandlungsergebnis in Relation zu den dafür aufgewendeten Kosten gesetzt wird. Eine entsprechende Berechnungsvorschrift kann bspw. in der folgenden Weise gestaltet sein:

$$\text{Outcome (E,K)} = \text{Outcome (Q)} : \text{Kosten} = E : K,$$

wobei das Ergebnis E in allgemeinster Form als prozentuale Verbesserung eines zu Behandlungsbeginn bestehenden Defizits gemessen wird und die Kosten K alle für die Erreichung des Ziels eingesetzten Ressourcen umfassen. Bei dieser bevorzugten Weiterbildung des vorliegenden Verfahrens werden somit. neben den Maßzahlen für die Bewertung des Defizits auch Maßzahlen für die im Rahmen der Therapie anfallenden Kosten berücksichtigt. Durch die Angabe des kostenorientierten Outcomes (E,K) in relativen Einheiten E/K, d. h. pro Währungseinheit, statt in absoluten Einheiten, lässt sich dieser kostenorientierte Behandlungserfolg auch über mehrere Teilziele hinweg oder über mehrere Gesundheitsinstitutionen bzw. Leistungserbringer hinweg leicht miteinander vergleichen.

In einer weitergebildeten Ausführungsform des Verfahrens werden die Kosten in nicht beeinflussbare Kostenanteile, und in Kostenanteile unterschieden, die stark variabel und durch Maßnahmen des Leistungserbringers oder durch Verhaltensweisen des Patienten beeinflussbar sind. Nicht beeinflussbare Kostenanteile sind bspw. Fixkosten, die bei jeder Behandlung in gleichem Maße anfallen sowie Overhead-Kosten, die bspw. proportional zur Behandlungsdauer anfallen, aber immer in etwa gleich hoch sind. Derartige nicht beeinflussbare Kostenanteile müssen nicht oder nur als grober, fester Schätzwert K (fix) berücksichtigt werden, während die beeinflussbaren Kostenanteile K (var) konkret erfasst werden. Der kostenorientierte Behandlungserfolg Outcome (E,K) kann hierbei entweder nur in Abhängigkeit von den beeinflussbaren Kostenanteilen Outcome (E,K) = Ergebnis : K (var) oder in Abhängigkeit von beiden Kostenanteilen Outcome (E,K) = Ergebnis : (K (fix) + K (var)) angegeben werden. Durch diese Vorgehensweise wird der Aufwand zur Erhebung der erforderlichen Maßzahlen minimiert, ohne dass die Aussagekraft des resultierenden Behandlungserfolges darunter leidet. Eine Erhebung aller direkten und indirekten Kosten für eine Rehabilitationsmaßnahme wäre in der Realität viel zu aufwendig und ist für ein aussagekräftiges Ergebnis im Allgemeinen auch nicht erforderlich, da die Fixkosten durch den Leistungserbringer in der Regel nicht oder nur kaum beeinflussbar sind.

In einer weiteren vorteilhaften Ausgestaltung des vorliegenden Verfahrens ist vorgesehen, für die Berechnung des kostenorientierten Behandlungserfolges nicht tatsächliche Kosten zu erfassen, da diese im typischen Workflow eines klinischen Prozesses selten unmittelbar erfasst werden und somit auch nicht ohne erheblichen Mehraufwand zur Verfügung stehen. Anstatt der tatsächlichen Kosten werden vielmehr beim vorliegenden Verfahren Schlüsselgrößen für die Berechnung der Kosten erfasst, die einerseits Bestandteil des Behandlungs-Workflows sind und andererseits aber in hohem Maße proportional zu relevanten Kostenanteilen, insbesondere zu den dominanten, stark variablen und beeinflussbaren Kosten. Beispiele für derartige Schlüsselgrößen sind bspw. die Anzahl oder die Zeitdauer persönlicher Sitzungen mit dem Patienten, die in direktem Zusammenhang mit den dadurch verursachten Kosten stehen. Weitere Beispiele sind in einem der Ausführungsbeispiele am Ende der vorliegenden Beschreibung angegeben. Diese leicht erfassbaren Schlüsselzahlen können je nach erwarteter Aussagekraft des kostenorientierten Behandlungserfolges für die relevanten variablen Kostenanteile entweder direkt in die Berechnung des Outcome eingehen oder mit Wichtungsfaktoren W(i) versehen werden, der die jeweiligen Schlüsselfaktoren S(i) in Währungseinheiten umrechnet. K (var) = W(i) x S(i). Dies kann selbstverständlich sowohl für fixe als auch für variable Kostenanteile durchgeführt werden. Damit gilt allgemein:

$$\text{Outcome (E,K) = E : K}$$

$$\texttt{Outcome (E,K) = Ergebnis : (K (fix) + K (var)), mit:}$$
$$\texttt{K} = \sum_{j=1}^{n}(\texttt{Wfix(j) x Sfix(j))} + \sum_{i=1}^{n}(\texttt{Wfix(i) x Sfix(i)).}$$

Vorzugsweise werden zur Vereinfachung der Handhabung die ohnehin fixen Kostenanteile bei der Berechnung vernachlässigt oder durch einen groben Schätzwert ersetzt und nur wenige relevante und leicht automatisiert messbare Schlüsselzahlen S(i) mit zugehörigen Wichtungsfaktoren W(i) bei der Berechnung berücksichtigt. Grundsätzlich müssen hierbei zur Erstellung einer für einen Rehabilitationsprozess geeigneten Berechnungsvorschrift zur Berechnung des Outcome (E,K) zunächst die fixen und die variablen Kostenanteile des betrachteten Rehabilitationsprozesses identifiziert und in ihrer Größenordnung abgeschätzt werden. Sind Kostenanteile deutlich geringer als die Gesamtkosten, so können diese zugunsten der Vereinfachung und entsprechend der erforderlichen Genauigkeit vernachlässigt werden.

In einer weiteren Ausführungsform des vorliegenden Verfahrens wird weiterhin die für die Therapie insgesamt benötigte Zeit in die Berechnung einbezogen, die für das Erreichen des Therapieergebnisses erforderlich ist. Bei gleicher Qualität des Ergebnisses und gleichen Kosten in kürzerer Zeit wird somit ein besserer Outcome (E,K,T) erreicht als bei längerer Therapiedauer. Daher gilt beispielhaft:

$$\text{Outcome (E,K,T) = Ergebnis : (Kosten x Zeit) = E : (K x T).}$$

Die Erfassung der bereits abgelaufenen Behandlungsdauer ist auf einfache Weise automatisiert möglich, bspw. durch Differenzbildung des Datums des Berechnungstages des Outcome zum Datum der ersten Verschreibung bzw. des Beginns der Therapie bzw. des Trainingsprogramms.

In einer weiteren Ausführungsform des vorliegenden Verfahrens wird in der Messung des Behandlungserfolges auch die Compliance C des Patienten, d. h. dessen Bereitschaft und Fähigkeit, die Therapiemaßnahmen wie verordnet durchzuführen, berücksichtigt. Ein Beispiel für eine Erfassung einer Maßzahl für die Compliance eines Patienten insbesondere in einem telemedizinischen Behandlungsprozess ist aus der DE 101 36 759.7 bekannt. Auch hier kann die Compliance durch eine Maßzahl, vorzugsweise in relativen Einheiten, bspw. in Prozent der gemäß Verschreibung erbrachten Leistungen, angegeben und während der Durchführung des Trainingsprogramms automatisiert erfasst werden.

Eine Berücksichtigung der Compliance des Patienten in einer Messung des Outcome einer Therapie ist aus den folgenden Gründen sinnvoll. Auf der einen Seite ist ein Benchmark für die Effizienz eines medizinischen Leistungserbringers ungerecht, wenn Faktoren einbezogen sind, auf die der Leistungserbringer keinen Einfluss hat. Ein derartiger Faktor ist die Kooperationsbereitschaft des Patienten, die in Form der Maßzahl der Compliance ausgedrückt wird. Eine mangelnde Compliance des Patienten muss also ein schlechtes Outcome-Ergebnis zum Teil zugunsten des Leistungserbringers kompensieren. Dies kann bspw. durch folgende Berechnungsvorschrift erreicht werden:

$$\text{Outcome (E,K,T,C)} = \text{Ergebnis} : (\text{Kosten} \times \text{Zeit} \times \text{Compliance}).$$

Bei diesem Beispiel einer Berechnungsvorschrift ergibt sich bei 100 % Compliance (C = 1) der Outcome in gleicher Weise wie ohne Berücksichtigung der Compliance. Ist die Compliance des Patienten schlecht, so wird der Outcome zwar sinken, aber mittels der Division durch eine Zahl C < 1 rechnerisch wieder angehoben. Andererseits kann es gerade ein Teil der Aufgabe und damit der Verantwortung des medizinischen Leistungserbringers sein, die Compliance des Patienten durch geeignete Maßnahmen sicherzustellen bzw. zu verbessern. Dies ist häufig bei sog. Disease Management Services für chronische Krankheiten wie bspw. Diabetes oder Asthma der Fall. Bei derartigen Therapien muss eine schlechte Compliance des Patienten als mindernd für den Outcome in die Berechnung einbezogen werden, wobei die Maßzahl C für die Compliance im Zähler der Berechnungsformel steht oder dem Outcome hinzugezählt wird:

$$\text{Outcome} \sim E \times C, \text{ oder Outcome} \sim E + C.$$

Eine niedrige Compliance C < 1 verringert hierbei den Outcome. Diese Form der Berechnung bestraft den Leistungserbringer dafür, dass es ihm nicht gelungen ist, den Patienten zu motivieren. Dabei wird implizit angenommen, dass das Ergebnis bei höherer Compliance besserer ausgefallen wäre. Gegebenenfalls müssen die Werte für Outcome und Compliance auch getrennt analysiert werden. So muss z. B. bei sehr schlechtem Ergebnis, d. h. E nahe Null, aber guter Comliance, d. h. C nahe 1, der Schluss gezogen werden, dass die Behandlungsstrategie falsch ist.

Bei dem vorliegenden Verfahren werden für unterschiedliche Therapien geeignete Berechnungsvorschriften vorzugsweise bereits durch Anbindung der ersten Datenverarbeitungsstation an eine Datenbank mit diesen Informationen bereitgestellt. Das Gleiche gilt für einzelnen Therapien zugeordnete Kostenanteile, aufgeteilt in beeinflussbare und nicht beeinflussbare Kosten sowie in zugehörige Schlüsselgrößen, ggf. mit den entsprechenden Wichtungsfaktoren. Dem Benutzer steht hierbei eine computerbasierte Benutzeroberfläche zur Verfügung, mit der er in der Auswahl der Maßzahlen und der Definition oder Auswahl der Berechnungsvorschrift für die Berechnung des Outcome unterstützt wird. Die Auswahl der Berechnungsvorschrift sowie der zugehörigen Kategorien erfolgt hierbei vorzugsweise durch Computer-übliche Eingabemechanismen, wie beispielsweise Mausklick, Drag-and-Drop etc.. Die erstellte bzw. gewählte Berechnungsvorschrift wird dabei automatisch in einem Speicher abgelegt und die Zugriffe auf die für die Berechnung des Outcome erforderlichen Daten werden durch die Datenverarbeitungsstation vorgenommen. Schließlich kann durch ein derartiges Computermodul auch eine automatisierte Konfiguration der zweiten Datenverarbeitungsstation für einen heimbasierten Trainings-Arbeitsplatz für den Patienten zur Durchführung der Rehabilitationsmaßnahmen mit Software- und Hardware-basierten Mitteln erfolgen, die die Mittel zur Erhebung der für die Outcome-Berechnung notwendigen Maßzahlen bereitstellt.

Das vorliegende System zur Durchführung des Verfahrens besteht aus einer ersten Datenverarbeitungsstation für den Benutzer bzw. Therapeuten sowie zumindest einer zweiten Datenverarbeitungsstation an dem Ort, an dem die Therapiemaßnahmen durchgeführt werden. Die zweite Datenverarbeitungsstation steht dabei vorzugsweise im häuslichen Umfeld des Patienten, so dass dieser die ihm verordneten Therapiemaßnahmen im häuslichen Umfeld durchführen kann. Erste und zweite Datenverarbeitungsstation sind zumindest zeitweise zum Datenaustausch miteinander vernetzt. In der ersten Datenverarbeitungsstation ist ein erstes Programmmodul zur interaktiven Festlegung des Behandlungsziels und zum Bereitstellen einer Berechnungsvorschrift für den Behandlungserfolg der Therapie in Bezug auf das Behandlungsziel vorgesehen. Das erste Modul hat Zugriff auf eine oder mehrere Datenbanken, in denen entsprechende Berechnungsvorschriften und Maßzahlen für verschiedene Therapien abgespeichert sind. Weiterhin ist das erste Modul derart ausgebildet, dass es eine Verbindung zu einem zweiten Modul in der zweiten Datenverarbeitungsstation herstellen und dieses zur Erfassung bestimmter Maßzahlen anweisen kann. Das zweite Modul ist hierbei derart ausgestaltet, dass es die Erfassung der vom ersten Modul angeforderten Maßzahlen durchführt oder koordiniert und diese Maßzahlen an das erste Modul übermittelt.

Das vorliegende Verfahren sowie das zugehörige System ermöglichen eine automatisierte und zuverlässige Messung des Behandlungserfolges einer medizinischen Therapie in standardisierten, relativen Einheiten, insbesondere in der Rehabilitation oder dem Disease Management chronischer Krankheiten. Das Verfahren und das System lassen sich in einfacher Weise in den üblichen klinischen Arbeitsablauf bzw. den Disease Management Service einbetten. Hierfür wird eine Datenvernetzungsstruktur mit zumindest zwei Datenverarbeitungsstationen eingesetzt, die die Daten zur automatisierten Berechnung im Therapieverlauf auch in einem räumlich verteilten Behandlungsprozess, insbesondere in einem telemedizinischen Therapieprozess oder einer telemedizinischen Rehabilitationsmaßnahme, bereitstellen. Bei mehrfachen Messungen während der Behandlung wird gleichzeitig eine quantifizierte Therapieverlaufskontrolle durch kontinuierliches Monitoring des Outcome-Verlaufs ermöglicht. Bei signifikanten Abweichungen dieses Outcome-Verlaufs von einer vorgebbaren Referenzkurve kann auch eine Meldung an den Benutzer, d. h. den betreuenden Arzt oder Therapeuten, gesendet werden, um möglicherweise einen Abbruch oder eine Änderung der Therapie herbeizuführen. In einer besonders vorteilhaften Ausführungsform erfolgt eine kostenorientierte Messung des Therapieerfolges, die unter dem zunehmenden Kostendruck im Gesundheitswesen eine besondere Aussagekraft besitzt. Das Verfahren umfasst die Bereitstellung einer Infrastruktur aus Datenbanken und einer Computerarbeitsplatz-Vernetzung zur Erhebung der für die Berechnung des Outcome benötigen Daten im Rahmen eines räumlich verteilten Therapieprozesses.

Das vorliegende Verfahren sowie das zugehörige System werden nachfolgend anhand von Ausführungsbeispielen in Verbindung mit den Zeichnungen ohne Beschränkung des allgemeinen Erfindungsgedankens nochmals erläutert. Hierbei zeigen:

Fig. 1     ein Beispiel für die Infrastruktur aus Datenbanken und Datenverarbeitungsstationen mit den darin ausgeführten Verfahrensschritten; und

Fig. 2     ein Beispiel für Möglichkeiten zur Erfassung der für eine Berechnungsvorschrift erforderlichen Maßzahlen an der zweiten Datenverarbeitungsstation.

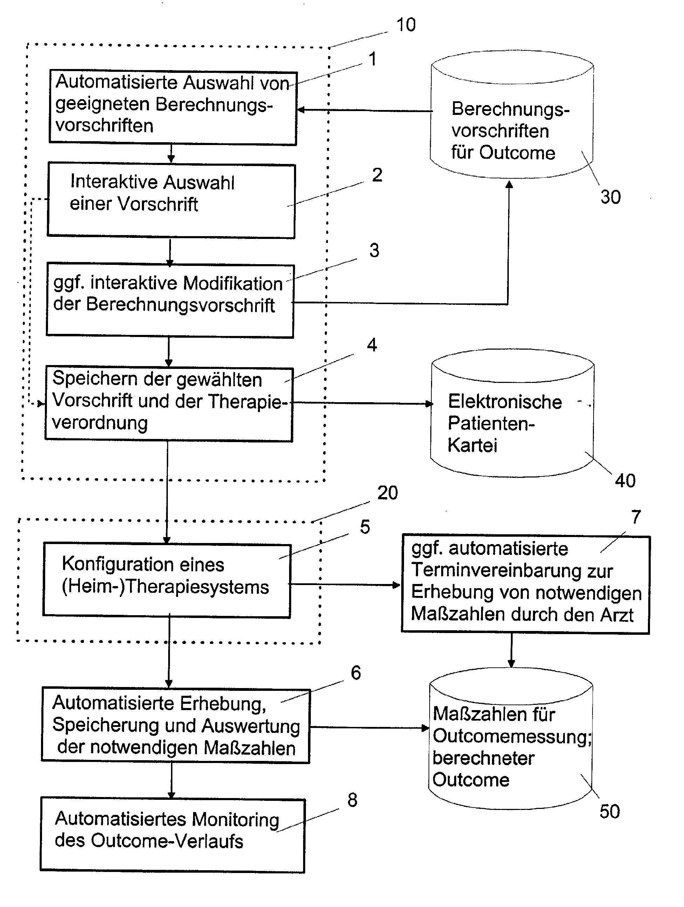

Figur 1 zeigt beispielhaft einzelne Verfahrensschritte für die Durchführung des vorliegenden Verfahrens gemäß einer möglichen Ausführungsform sowie die zugehörigen Datenverarbeitungsstationen sowie Datenbanken. Das Verfahren beginnt an einem Computerarbeitsplatz (erste Datenverarbeitungsstation 10) zur Therapieplanung und Therapieverlaufskontrolle. Dieser Computerarbeitsplatz 10 beinhaltet ein Softwaremodul, das den Benutzer darin unterstützt, zu Beginn der Behandlung ein geeignetes Outcome-Berechnungsschema für den dem Patienten verordneten Therapieprozess zusammenzustellen, diesem Therapieprozess zuzuordnen und Mittel für die automatisierte Erfassung und Auswertung der hierfür notwendigen Messdaten bereitzustellen. Hierfür sind im vorliegenden Beispiel auf dem Computerarbeitsplatz 10 ein oder mehrere Datenbanken 30 abgespeichert, die für jede Therapie eine Liste der relevanten Kostenblöcke enthält. Diese Kosten sind bereits vorklassifiziert in fixe und variable Kosten gemäß der vorangegangenen Definition. Weiterhin enthält die Datenbank 30 für jeden Kostenblock eine Maßzahl für die Größe der Kosten, bspw. in Währungseinheiten. Diese Maßzahlen können als Voreinstellung in der Datenbank abgelegt sein, jedoch auch individuell an die Gegebenheiten des jeweiligen Leistungserbringers bzw. der jeweiligen Institution angepasst werden. Weiterhin existiert eine Datenbank mit Schlüsselgrößen für die Messung von Kostenblöcken und deren Zuordnung zum Kostenblock. Die Datenbank kann auch noch jeder Schlüsselgröße zugeordnete Wichtungsfaktoren enthalten, die eine Umrechnung der Schlüsselgrößen in Währungseinheiten erlaubt.

Als vereinfachtes Beispiel soll im Folgenden ein Patient mit kognitiven Störungen nach einem Schlaganfall angenommen werden, der durch ein telemedizinisch betreutes Trainingsprogramm, das zu Hause an einem Computertrainingsplatz (zweite Datenverarbeitungsstation 20) durchgeführt wird, behandelt werden soll. Bei diesem Beispiel sind fixe und kaum beeinflussbare Kostenanteile die einmalige Bereitstellung des Computertrainingsplatzes 20 und die Einweisung des Patienten, die Erstuntersuchung und Einstufung des Patienten hinsichtlich seines Defizits, die erste Zusammenstellung, Verschreibung und Übermittlung eines individuell optimalen Trainingsprogramms. Relevante variable Kostenanteile für die Berechnung eines kostenorientierten Outcomes sind dagegen die persönlichen Sitzungen mit dem Patienten, die Auswertung von Alarm- und Informationsmeldungen aus den Trainingssitzungen, die Anzahl der routinemäßigen Beurteilungen der Trainingsergebnisse und des Trainingsfortschritts, die Anpassung und Neuverschreibung des Trainingsprogramms sowie die Wiedereinweisung des Patienten oder ambulante Behandlungen. Geeignete messbare bzw. zählbare Schlüsselgrößen $S(i)$ für die variablen Kostenanteile dieses Rehabilitationsprozesses sind daher die Dauer der Behandlung, die Anzahl der Arzt/Therapeuten-Konsultationen, die Anzahl der Krankenhaustage in einer Rehaklinik, die Anzahl der Alarm- oder Informations-Meldungen, die Anzahl der manuellen Trainingsprogramm-Modifikationen durch den Arzt oder Therapeuten, die Anzahl der Login's des Arztes bzw. Therapeuten in einen Patienten-Account bzw. die Anzahl der Zugriffe auf die elektronische Patientenakte des Heimtherapiesystems, sowie gegebenenfalls die Anzahl der übertragenen Daten zwischen Heim und Klinik bzw. Praxis bei einem datenmengenbezogenen Abrechnungsmodus des Serviceproviders. Nicht relevant für die Gesundheitskosten und damit für die Messung ungeeignet ist bspw. die Zeit, die der Patient alleine mit der Durchführung des Trainings verbringt, und damit die Anzahl der durchgeführten Trainingseinheiten. Diese Schlüsselzahlen S(i) lassen sich sehr einfach automatisiert erfassen, so dass die Einbeziehung der Kosten in das Verfahren keinen größeren Aufwand verursacht.

In einer Benutzeroberfläche des Computerarbeitsplatzes 10 sind für den Anwender, d. h. den Arzt oder Therapeuten, nach Auswahl einer Therapie bzw. eines Trainingsprogramms automatisch alle für dieses Programm relevanten Kostenblöcke dargestellt, die nicht relevanten Kostenblöcke sind ausgeblendet. Optional kann für alle Kostenblöcke graphisch deren relativer Anteil an den Gesamtkosten, bspw. in Form eines Balkendiagramms, dargestellt werden. Der Anwender kann nun mit den üblichen einfachen interaktiven Bedienungsmitteln, d. h. Mausklick, Ankreuzen in einer Liste, drag & drop usw., diejenigen Kostenblöcke markieren, die er in die Berechnung des Outcome einbeziehen will. Zusätzlich kann der Benutzer markieren, welche Kategorien neben dem Behandlungsergebnis, d. h. Kosten, Behandlungsdauer und Compliance, in die Berechnung des Outcome einbezogen werden sollen. Mit den ausgewählten Vorgaben als Randbedingung wird dem Anwender nun eine Auswahl möglicher Berechnungsvorschriften angezeigt, die in der Datenbank 30 in Bezug auf die jeweiligen Therapien und Kategorien abgespeichert sind. Aus dieser Auswahl der möglichen Berechnungsvorschriften kann der Benutzer wiederum eine für ihn geeignet erscheinende markieren.

Wahlweise kann die Bedienung auch in anderer Reihenfolge erfolgen, wobei dem Anwender automatisch eine Liste von für das ausgewählte Trainingsprogramm relevanten Outcome-Berechnungsvorschriften angezeigt wird (Schritt 1), aus denen er eine auswählt (Schritt 2). In der Folge erhält der Anwender dann eine Übersicht über alle Maßzahlen, die künftig für die Berechnung dieses Outcome mit der gewählten Berechnungsvorschrift erhoben und ausgewertet werden. Optional kann vorgesehen sein, dass der Anwender die ausgewählte Berechnungsvorschrift durch Auswahl eines zugeordneten Satzes von Maßzahlen noch weiter spezifiziert.

In einer weiteren möglichen Ausgestaltung des Verfahrens kann der Anwender die Berechnungsvorschriften für den Behandlungserfolg editieren, d. h. neu erstellen oder verändern, und die neu erstellten Berechnungsvorschriften der Datenbank 30 möglicher Berechnungsschemata hinzufügen (Schritt 3).

Nachdem sich der Anwender im Schritt 2 für eine Berechnungsvorschrift des Outcome entschieden hat, wird diese unter Zuordnung zum Patienten und dem gewählten Trainingsprogramm abgespeichert (Schritt 4). Diese Daten werden in einer Datenbank 40 oder einer elektronischen Patientenkartei (EPR) abgespeichert. Anschließend erstellt der Computer 10 selbsttätig die Zugriffe (Verweise auf Dateien usw.) auf die für die Berechnung notwendigen Maßzahlen. Das Programmmodul kann auch automatisiert Maßnahmen zur Bereitstellung aller Mittel einleiten, die zur Messung der im Therapieverlauf erforderlichen Maßzahlen notwendig sind. Dies kann bspw. die Freischaltung dafür vorgesehener Messmodule in der patientenseitigen Trainingssoftware (zweites Programmmodul) an der zweiten Datenverarbeitungsstation 20 (Computertrainingsplatz), wie bspw. Lebensqualitäts-Fragebogen, computerbasierte Fähigkeitstests, usw. umfassen. Bei dem vorliegenden Verfahren erfolgt nach Festlegung des Trainingsprogramms sowie der Berechnungsvorschrift auch die Konfiguration des beim Patienten zu installierenden Computertrainingsplatzes vorzugsweise automatisch, etwa durch automatische Spezifikation der mitzuliefernden Sensormodule (Schritt 5). Dies schließt auch die Erstellung eines Zeitplanes zur Messung der Schlüsselgrößen sowie das automatische Anstoßen des Messvorganges für die Messung der Maßzahlen für den Behandlungserfolg ein. Vorzugsweise wird hierbei vom Programmmodul der ersten Datenverarbeitungsstation 10 in regelmäßigen Zeitabständen oder zu vorgegebenen Messzeitpunkten geprüft, ob neue Maßzahlen vorliegen. Alternativ kann bei Entstehung neuer Maßzahlen ein Hinweis an das Programmmodul versendet oder manuell interaktiv am Bildschirm eine neue Berechnung des Outcome angestoßen werden. Die Maßzahlen für die Berechnung des Behandlungserfolges werden automatisch am Computertrainingsplatz des Patienten 20 erhoben, gespeichert und von der ersten Datenverarbeitungsstation entsprechend der gewählten Berechnungsvorschrift ausgewertet (Schritt 6). Die im Verlauf der Behandlung erhobenen Maßzahlen sowie Outcome-Werte werden in einer Datenbank 50 abgespeichert und sind jederzeit für eine Darstellung, bspw. in graphischer Form oder in Form einer Auflistung, am Computerarbeitsplatz 10 verfügbar. Der Outcome-Verlauf über die Zeit wird automatisch am Bildschirm angezeigt (Schritt 8). Gegebenenfalls kann bei signifikanten Abweichungen von einer Referenzkurve auch eine Alarmgebung an einer der beteiligten Datenverarbeitungsstationen 10, 20 erfolgen.

Benötigt das Outcome-Berechnungsprogramm Maßzahlen, die nicht automatisiert im Verlauf der Therapiedurchführung erhoben werden können, so kann das Programm automatisch einen Termin mit den zuständigen Personen, bspw. Arzt und Patient, zu vorgegebenen Zeitpunkten koordinieren, an dem die notwendigen Maßzahlen, bspw. durch Messung eines Fähigkeitsdefizits in einer Rehaklinik, erhoben werden (Schritt 7).

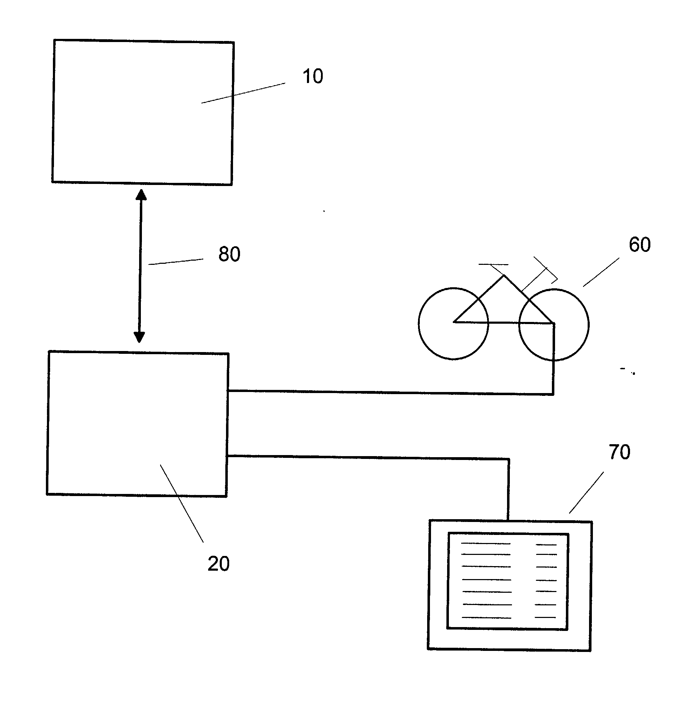

Figur 2 zeigt ein Beispiel für eine Vernetzung der beiden Datenverarbeitungsstationen 10, 20 über ein Netzwerk 80, wobei die häusliche Datenverarbeitungsstation 20 des Patienten in diesem Beispiel über eine Schnittstelle zusätzlich mit einem Ergometer 60 verbunden ist, über das Maßzahlen über eine Leistungsfähigkeit des Patienten erhoben werden können. Weiterhin ist in dieser Figur die Erhebung von Maßzahlen über einen Fragebogen 70 veranschaulicht, der am Bildschirm des Computertrainingsplatzes zum erforderlichen Messzeitpunkt angezeigt wird, um den Patienten zur Bearbeitung zu veranlassen. Durch diesen Computertrainingsplatz 20 wird dem Patienten auch das im Rahmen der Therapie zu absolvierende Trainingsprogramm vorgegeben.

Der Nutzen des vorliegenden Verfahrens sowie des zugehörigen Systems erschließt sich insbesondere bei der Behandlung mittels innovativen telemedizinischen Behandlungsformen und der Betreuung des Patienten zu Hause (Homecare). Einige Teilaspekte des vorliegenden Verfahrens sind auf derartige Anwendungen besonders abgestimmt. So ist die geeignete Konfiguration eines heimbasierten Trainings-Arbeitsplatzes zur Erhebung von für die Outcome-Berechnung notwendigen Maßzahlen eine zwingende Voraussetzung, um mit logistisch vertretbarem Aufwand eine Outcome-Messung bei telemedizinischer Rehabilitation durchführen zu können.

**Patentansprüche**

1. Verfahren zur Messung des Behandlungserfolges einer medizinischen Therapie mit folgenden Schritten:

   - Festlegung zumindest eines Behandlungsziels der Therapie an einer ersten Datenverarbeitungsstation (10);
   - Bereitstellen oder Eingeben einer Berechnungsvorschrift für den Behandlungserfolg der Therapie in Bezug auf das Behandlungsziel, die den Behandlungserfolg als Funktion erfassbarer Maßzahlen angibt, an der ersten Datenverarbeitungsstation (10);
   - Erfassen einer oder mehrerer den Ausgangszustand einer der Therapie zu unterziehenden Person in Bezug auf das Behandlungsziel beschreibenden ersten Maßzahlen;
   - Abspeichern von Daten, die zumindest das Behandlungsziel., die Berechnungsvorschrift und die ersten Maßzahlen umfassen;
   - Erfassen einer oder mehrerer den Behandlungserfolg der Person beschreibenden zweiten Maßzahlen während und/oder am Ende der Therapie durch eine zweite Datenverarbeitungsstation (20) ;
   - Abrufen der abgespeicherten Daten und automatische Berechnung des Behandlungserfolges mit der Berechnungsvorschrift an der ersten (10) oder zweiten Datenverarbeitungsstation (20), wobei die zweiten Maßzahlen bzw. der gegebenenfalls bereits an der zweiten Datenverarbeitungsstation (20) berechnete Behandlungserfolg über ein Netzwerk (80) an die erste Datenverarbeitungsstation (10) übermittelt werden; und
   - Darstellen des Behandlungserfolges an der ersten Datenverarbeitungsstation (10).

2. Verfahren zur Bestimmung des Behandlungserfolges einer medizinischen Therapie, insbesondere nach Anspruch 1, bei dem an einem Computerarbeitsplatz eine Berechnungsvorschrift bereitgestellt wird, die den Behandlungserfolg als eine Funktion der Kosten oder der Kosten und der Dauer der Therapie und/oder der Compliance des Patienten angibt.

3. Verfahren nach Anspruch 1 oder 2,
   **dadurch gekennzeichnet,**
   **dass** die Berechnungsvorschrift eine Vergleichsvorschrift ist, nach der erfasste Maßzahlen mit einer in einer Datenbank abgespeicherten Look-Up-Tabelle verglichen werden, welche die erfassten Maßzahlen mit einem Wert für den Behandlungserfolg verknüpft.

4. Verfahren nach einem der Ansprüche 1 bis 3,
   **dadurch gekennzeichnet,**
   **dass** das Bereitstellen der Berechnungsvorschrift die Auswahl aus zur Therapie zugeordneten Berechnungsvorschriften umfasst, die automatisch aus einer Datenbank (30) abgerufen werden.

5. Verfahren nach einem der Ansprüche 1 bis 3,
   **dadurch gekennzeichnet,**
   **dass** das Bereitstellen der Berechnungsvorschrift die Auswahl von Kategorien umfasst, die in die Berechnung einbezogen werden sollen, wobei die Kategorien und der Auswahl zugeordnete Berechnungsvorschriften aus einer Datenbank (30) abgerufen werden.

6. Verfahren nach Anspruch 5,
   **dadurch gekennzeichnet,**
   **dass** die Kategorien in der Datenbank (30) Behandlungszielen zugeordnet sind und nur die Kategorien zur Auswahl angeboten werden, die für das jeweilige Behandlungsziel relevant sind.

**7.** Verfahren nach Anspruch 5 oder 6,
**dadurch gekennzeichnet,**
**dass** die Auswahl der Berechnungsvorschrift sowie der zugehörigen Kategorien durch Computer-übliche Eingabemechanismen, insbesondere durch Mausklick oder Drag-and-Drop erfolgt.

**8.** Verfahren nach einem der Ansprüche 4 bis 7,
**dadurch gekennzeichnet,**
**dass** die Berechnungsvorschrift für den Benutzer editierbar bereitgestellt und eine durch den Benutzer neu erstellte oder geänderte Berechnungsvorschrift der Datenbank (30) hinzugefügt wird.

**9.** Verfahren nach einem der Ansprüche 1 bis 8,
**dadurch gekennzeichnet,**
**dass** die zweite Datenverarbeitungsstation (20) zur automatisierten Erfassung und Übermittlung der den Behandlungserfolg beschreibenden zweiten Maßzahlen an die erste Datenverarbeitungsstation (10) konfiguriert wird.

**10.** Verfahren nach einem der Ansprüche 1 bis 9,
**dadurch gekennzeichnet,**
**dass** das Erfassen der den Behandlungserfolg beschreibenden zweiten Maßzahlen von der ersten Datenverarbeitungsstation (10) veranlasst wird oder in vorgebbaren Zeitabständen erfolgt.

**11.** Verfahren nach einem der Ansprüche 1 bis 10,
**dadurch gekennzeichnet,**
**dass** ein oder mehrere der zweiten Maßzahlen über mit der zweiten Datenverarbeitungsstation (20) verbundene Mittel (60, 70) erfasst werden.

**12.** Verfahren nach einem der Ansprüche 1 bis 11,
**dadurch gekennzeichnet,**
**dass** durch die erste und/oder zweite Datenverarbeitungsstation (20) für die Erfassung von zweiten Masszahlen, die nicht automatisiert erhoben werden können, ein Hinweis, insbesondere ein Vorschlag für eine Terminvereinbarung zur Erhebung dieser zweiten Maßzahlen, generiert wird.

**13.** Verfahren nach einem der Ansprüche 1 bis 12,
**dadurch gekennzeichnet,**
**dass** an der ersten Datenverarbeitungsstation (10) Teilziele vorgegeben werden, deren Erreichen durch mehrfache Berechnung des Behandlungserfolges während der Dauer der Behandlung überwacht wird.

**14.** Verfahren nach Anspruch 13,
**dadurch gekennzeichnet,**
**dass** die zu verschiedenen Zeitpunkten gemessenen Behandlungserfolge abgespeichert und in Form einer Verlaufskurve dargestellt werden.

**15.** Verfahren nach Anspruch 14,
**dadurch gekennzeichnet,**
**dass** eine Referenzkurve für den Verlauf vorgegeben und bei Abweichen der Verlaufskurve von der Referenzkurve um einen vorgebbaren Wert eine Alarmmeldung erzeugt wird.

**16.** Verfahren nach einem der Ansprüche 1 bis 15,
**dadurch gekennzeichnet,**
**dass** der Behandlungserfolg in Bezug auf die dafür aufgewendeten Kosten berechnet wird.

**17.** Verfahren nach Anspruch 16,
**dadurch gekennzeichnet,**
**dass** bei den Kosten nur die durch den Leistungserbringer und/oder den Patienten beeinflussbaren Kosten in die Berechnung einbezogen werden.

**18.** Verfahren nach Anspruch 16 oder 17,
**dadurch gekennzeichnet,**
**dass** die Kosten mit Schlüsselgrößen berücksichtigt werden, die auf einfache Weise erfassbar und proportional zu den Kosten sind.

**19.** Verfahren nach Anspruch 18,
**dadurch gekennzeichnet,**
**dass** Kostenanteile und Schlüsselgrößen zu der gewählten Therapie von einer Datenbank abgerufen und automatisiert zur Auswahl angezeigt werden.

**20.** Verfahren nach einem der Ansprüche 1 bis 19,
**dadurch gekennzeichnet,**
**dass** die Berechnung des Behandlungserfolges in Bezug zu der für die Behandlung aufgewendeten Zeit erfolgt.

**21.** Verfahren nach einem der Ansprüche 1 bis 20,
**dadurch gekennzeichnet,**
**dass** die Berechnung des Behandlungserfolges unter Berücksichtigung der Compliance des Patients erfolgt.

**22.** System zur Durchführung des Verfahrens nach einem der Ansprüche 1 - 21, mit

- einer ersten Datenverarbeitungsstation (10) mit einem ersten Modul zur Festlegung des Behandlungsziels, zur Bereitstellung der Berechnungsvorschrift und zur automatisierten Durchführung der Berechnung anhand von übermittelten oder abgespeicherten Maßzahlen, und
- einer zweiten mit der ersten zumindest zeitweise über ein Netzwerk (80) verbundenen Datenverarbeitungsstation (20) mit einem zweiten Modul zur automatischen Erfassung und Übermittlung von Maßzahlen an das erste Modul der ersten Datenverarbeitungsstation zu vorgebbaren Zeiten.

**23.** System nach Anspruch 22,
**dadurch gekennzeichnet,**
**dass** das erste Modul zur Bereitstellung der Berechnungsvorschrift mit einer Datenbank (30) verbunden ist, die unterschiedlichen Therapien und Behandlungszielen zugeordnete Berechnungsvorschriften enthält.

**24.** System nach Anspruch 22 oder 23,
**dadurch gekennzeichnet,**
**dass** die zweite Datenverarbeitungsstation (20) über eine oder mehrere Schnittstellen mit Mitteln (60, 70) zur Messung der Maßzahlen verbunden ist.

10

80

60

70

20

Europäisches
Patentamt

**EUROPÄISCHER TEILRECHERCHENBERICHT**

der nach Regel 45 des Europäischen Patentübereinkommens für das weitere Verfahren als
europäischer Recherchenbericht gilt

**Nummer der Anmeldung**

EP 02 00 8042

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.7) |
|---|---|---|---|
| A | US 5 524 645 A (WILLS BRUCE R) 11. Juni 1996 (1996-06-11) * das ganze Dokument * --- | 1 | G06F19/00 |
| A | WO 96 30848 A (COX MICHAEL W ;LEVIN RICHARD I (US)) 3. Oktober 1996 (1996-10-03) * das ganze Dokument * --- | 1 | |
| A | WO 00 75853 A (UNIV OKLAHOMA STATE) 14. Dezember 2000 (2000-12-14) * das ganze Dokument * --- | 1 | |
| A | US 5 435 324 A (BRILL PETER L) 25. Juli 1995 (1995-07-25) * das ganze Dokument * --- | 1 | |
| A | US 5 582 186 A (WIEGAND RAYMOND A) 10. Dezember 1996 (1996-12-10) * das ganze Dokument * ----- | 1 | |

**RECHERCHIERTE SACHGEBIETE (Int.Cl.7)**

G06F
A61B

## UNVOLLSTÄNDIGE RECHERCHE

Die Recherchenabteilung ist der Auffassung, daß ein oder mehrere Ansprüche, den Vorschriften des EPÜ in einem solchen Umfang nicht entspricht bzw. entsprechen, daß sinnvolle Ermittlungen über den Stand der Technik für diese Ansprüche nicht, bzw. nur teilweise, möglich sind.

Vollständig recherchierte Patentansprüche:

Unvollständig recherchierte Patentansprüche:

Nicht recherchierte Patentansprüche:

Grund für die Beschränkung der Recherche:

Siehe Ergänzungsblatt C

| Recherchenort | Abschlußdatum der Recherche | Prüfer |
|---|---|---|
| BERLIN | 10. Oktober 2002 | Bernas, Y |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
....................................................................
& : Mitglied der gleichen Patentfamilie, übereinstimmendes Dokument

EPO FORM 1503 03.82 (P04C09)

## UNVOLLSTÄNDIGE RECHERCHE
### ERGÄNZUNGSBLATT C

Unvollständig recherchierte Ansprüche:
1-24

Grund für die Beschränkung der Recherche:

Die geltenden Patentansprüche 1-24 beziehen sich auf ein Verfahren bzw.
eine Vorrichtung, jeweils charakterisiert durch eine erstrebenswerte
Eigenheit oder Eigenschaft, nämlich die Automatisierung der Berechnung
eines Behandlungserfolges.
Die Patentansprüche umfassen daher alle Verfahren bzw. Vorrichtungen die
diese Eigenheit oder Eigenschaft aufweisen. Die Patentanmeldung liefert
dagegen überhaupt keine Offenbarung für die Art und Weise der Ausführung
dieser Funktionen und es ist auch nicht ersichtlich wie der Fachmann die
entschprechenden  Funktionen bewerkstelligen könnte ohne selbst
erfinderisch tätig zu werden. Es fehlt daher insgesamt  die Stütze durch
die Beschreibung im Sinne von Art. 83 EPÜ . Im vorliegenden Fall fehlen
den Patentansprüchen die entsprechende Stütze bzw. der Patentanmeldung
die nötige Offenbarung in einem solchen Maße, daß eine sinnvolle
Recherche über den gesamten erstrebten  Schutzbereich unmöglich
erscheint.
 Desungeachtet fehlt den Patentansprüchen auch die in Art. 84 EPÜ
geforderte Klarheit, nachdem in ihnen versucht wird, das Verfahren bzw.
die Vorrichtung über das jeweils erstrebte Ergebnis zu definieren. Auch
dieser Mangel an Klarheit ist dergestalt, dass er eine sinnvolle
Recherche über den gesamten erstrebten Schutzbereich unmöglich macht.

Darüberhinaus könnte  selbst ein Erfindungsgegenstand der genügend klar
definiert und durch die Offenbarung  der Beschreibung ausreichend
gestützt wäre als Folge von Art. 52(2)c EPÜ  nicht als Erfindung
eingesehen werden .

Die Recherche wurde  daher darauf beschränkt  eine Auswahl von
automatischen Verfahren zur Berechnung des Behandlungserfolgs anzugeben.

**ANHANG ZUM EUROPÄISCHEN RECHERCHENBERICHT
ÜBER DIE EUROPÄISCHE PATENTANMELDUNG NR.**                EP 02 00 8042

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-10-2002

| Im Recherchenbericht angeführtes Patentdokument | | Datum der Veröffentlichung | Mitglied(er) der Patentfamilie | | Datum der Veröffentlichung |
|---|---|---|---|---|---|
| US 5524645 | A | 11-06-1996 | AU | 5319896 A | 08-10-1996 |
| | | | WO | 9629006 A1 | 26-09-1996 |
| WO 9630848 | A | 03-10-1996 | AU | 5530996 A | 16-10-1996 |
| | | | EP | 0846296 A1 | 10-06-1998 |
| | | | WO | 9630848 A1 | 03-10-1996 |
| | | | US | 5724580 A | 03-03-1998 |
| WO 0075853 | A | 14-12-2000 | WO | 0075853 A1 | 14-12-2000 |
| | | | AU | 4331599 A | 28-12-2000 |
| US 5435324 | A | 25-07-1995 | BR | 9306936 A | 12-01-1999 |
| | | | CA | 2142906 A1 | 03-03-1994 |
| | | | EP | 0656761 A1 | 14-06-1995 |
| | | | WO | 9404072 A1 | 03-03-1994 |
| US 5582186 | A | 10-12-1996 | KEINE | | |

EPO FORM P0461

Für nähere Einzelheiten zu diesem Anhang : siehe Amtsblatt des Europäischen Patentamts, Nr.12/82